# EUROPEAN PATENT APPLICATION

(11) **EP 4 535 282 A1**
(43) Date of publication of application: **09.04.2025**
(21) Application number: 23201112.2
(22) Date of filing: 02.10.2023
(51) Int. Cl.: G06T 7/00, G16H 50/20

(54) **MOBILE DEVICE AND COMPUTER IMPLEMENTED METHOD FOR REAL-TIME RETINAL DIAGNOSIS, DATA PROCESSING APPARATUS, COMPUTER PROGRAM AND COMPUTER READABLE MEDIUM**

(71) Applicant: Deutsches Zentrum für Luft- und Raumfahrt e.V., 53227 Bonn (DE)
(72) Inventor: RITTER, Scott, 50678 Köln (DE); DRESCHER, Jürgen, 51147 Köln (DE); STERN, Claudia, 51147 Köln (DE); TERRANOVA, Franco, 54000 Nancy (FR); COWLEY, Adrian, 51147 Köln (DE)
(74) Representative: dompatent von Kreisler Selting Werner - Partnerschaft von Patent- und Rechtsanwälten mbB

(57) **Abstract**

Mobile device for real-time retinal diagnosis comprising a capturing module configured to capture a number V of images of a retina of a patient, an object detection module configured to calculate an object detection score for the presence of an optic disc and to calculate the location of the optic disc for each of the number V of images with a trained neural network for the detection of an optic disc, an extraction module configured to extract a partial image area of the optic disc in each of the images of the retina of the patient based on the previously calculated location of the optic disc in each of the images, a classification module configured to forecast the presence or absence of optic disc edema for each of the extracted partial image areas containing the optic disc with a trained convolutional neural network for the identification of optic disc edema, and a predictor module configured to calculate an overall prediction for the presence of optic disc edema for the patient.

## Description

The current invention provides a mobile device as well as a computer implemented method for real-time retinal diagnosis by using trained neural networks and/or trained convolutional neural networks.

In clinical ophthalmology, retinal images collected from mobile device cameras are useful for diagnosing common health problems such as glaucoma, diabetic retinopathy, head injuries, and macular degeneration. These images are time consuming to analyze, and currently require a clinical specialist to carry out the diagnosis. Furthermore, less than 50% of patients with retinal pathologies have access to these specialists and/or eye fundus diagnostics, which puts them at risk of worsening vision. Blindness occurs in 10% of these cases (World Health Organization, 2019).

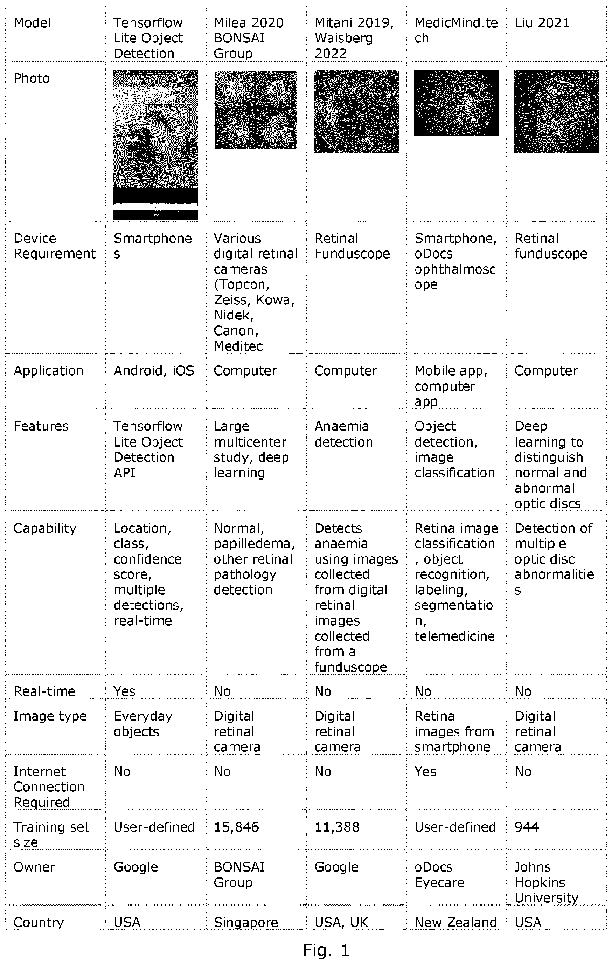

There are many free and commercially available artificial intelligence applications that use mobile device cameras for object detection and classification. In addition, there are many free and commercially available artificial intelligence applications that are used for optic disc edema. Examples of artificial intelligence application for mobile devices and the detection of optic disc edema are shown in the table of Figure 1.

The current invention provides a mobile device as well as a computer implemented method which uses real-time artificial intelligence models which help the user to navigate, identify and classify the optic disc as being normal or pathologic (absence or presence of optic disc edema). The aforementioned mobile device as well as the computer implemented method help non-specialist clinicians as well as normal users without a medical background to detect optic disc pathologies which non-specialist clinicians might otherwise miss, or which normal users without a medical background may be unable to identify.

The mobile devices as well as the computer implemented method according to the present invention can have a plurality of application scenarios and can be used for example in emergency medical services such as an emergency medical room, in spaceflight, but also in remote regions on Earth without access or without immediate access to specialist clinicians. The methods and/or mobile devices according to the present invention enable a real-time mobile diagnosis and thereby enable a therapy optimization in for example home care and emergency medicine scenarios.

The most important solutions on the market today have capabilities in artificial intelligence. The oDocs company provides artificial intelligence applications for its users. Many companies offer AI services for detecting common eye problems, but none of the state of the art methods and/or devices offer real-time diagnostics directly on the mobile device, with navigation, detection, and classification, without the need for an Internet connection of the according device or method. AI products are accurate and well developed, but lack proprietary datasets on which real-time, mobile device compatible models are developed.

In emergency medicine and aerospace medicine, retinal images are becoming useful indicators for increased intracranial pressure (ICP) and Spaceflight Associated Neuro-ocular Syndrome (SANS), which can be associated with optic disc edema, and may also lead to worsening vision and blindness (Mader, et al., 2011). But analyzing these retinal images is a laborious task for qualified ophthalmologists and specialized personnel, both on Earth and in space.

For example, during a recent technology demonstration during spaceflight, all retinal images had to be downloaded from the International Space Station (ISS) to clinical specialists for remote analysis. The downlink took several hours, and the analysis took several weeks. Artificial intelligence applications installed natively on a mobile device could greatly reduce the need for download to Earth and analysis by clinical specialists. This possibility is appealing for exploration class space missions in which crew members would not have real-time, continuous communication with Earth, and would need to operate with increased autonomy. This possibility is also appealing for remote medicine applications on Earth, where smartphones with cameras exist, but clinical specialists and an Internet connection may not.

The relevant literature references are listed below:
[1] De Fauw, J., et al., 2017. Automated analysis of retinal imaging using machine learning techniques for computer vision. F1000Research, 5:1573.
[2] De Fauw, J., et al., 2018. Clinically applicable deep learning for diagnosis and referral in retinal disease. Nature Medicine, 24, pp.1342-50.
[3] Dunn, H. P., Kang, C. J., Marks, S., Witherow, J. L., Dunn, S. M., Healey, P. R., & White, A. J. (2021). Perceived usefulness and ease of use of fundoscopy by medical students: a randomised crossover trial of six technologies (eFOCUS 1). BMC medical education, 21(1), 41.
[4] Dunn, H. P., Marks, S., Teo, K. Z., Dunn, S. M., Healey, P. R., & White, A. J. (2021). eFOCUS 2: A randomised crossover trial of smartphone fundoscopy and direct ophthalmoscopy aiming to improve optic disc interpretation by medical students with e-learning support. Clinical & experimental ophthalmology, 49(7), 704-713.
[5] European Space Agency, 2021. Artificial Intelligence for eye health in space. [online] Available at: <https://www.esa.int/About_Us/EAC/Artificial_intelligence for eye_h ealth_in_space>
[6] European Space Agency, 2022. Eye on world health. [online] Available at: <https://www.esa.int/ESA_Multimedia/Images/2022/04/Eye_on_worl d_health>
[7] He, G., Dunn, H. P., Ahmad, K. E., Watson, E., Henderson, A., Tynan, D., Leaney, J., White, A. J., Hewitt, A. W., & Fraser, C. L. (2022). Fundoscopy use in neurology departments and the utility of smartphone photography: a prospective prevalence and crossover diagnostic accuracy study amongst neurology inpatients. European journal of neurology, 29(8), 2463-2472.
[8] Liu, T Y Alvin et al. "Detection of Optic Disc Abnormalities in Color Fundus Photographs Using Deep Learning." Journal of neuro-ophthalmology : the official journal of the North American Neuro-Ophthalmology Society vol. 41,3 (2021): 368-374. doi: 10.1097/WNO.0000000000001358
[9] Milea, D., Najjar, R. P., Zhubo, J., Ting, D., Vasseneix, C., Xu, X., Aghsaei Fard, M., Fonseca, P., Vanikieti, K., Lagrèze, W. A., La Morgia, C., Cheung, C. Y., Hamann, S., Chiquet, C., Sanda, N., Yang, H., Mejico, L. J., Rougier, M.-B., Kho, R., Thi Ha Chau, T., ... BONSAI Group (2020). Artificial Intelligence to Detect Papilledema from Ocular Fundus Photographs. The New England Journal of Medicine, 382(18), 1687-1695.
[10] Mitani, Akinori et al. "Detection of anaemia from retinal fundus images via deep learning." Nature biomedical engineering vol. 4,1 (2020): 18-27. doi: 10.1038/s41551-019-0487-z
[11] Rajalakshmi, R., Subashini, R., Anjana, R.M., Mohan, V., 2018. Automated diabetic retinopathy detection in smartphone-based fundus photography using artificial intelligence. Eye, 32(6), pp.1138-1144.
[12] Waisberg, E., Ong, J., Zaman, N., Kamran, S. A., Lee, A. G., & Tavakkoli, A. (2022). A non-invasive approach to monitor anemia during long-duration spaceflight with retinal fundus images and deep learning. Life sciences in space research, 33, 69-71. https://doi.org/10.1016/j.lssr.2022.04.004
[13] Wintergerst, M.W.M., Petrak, M., Li, J.Q., Larsen, P.P., Berger, M., Holz, F.G., Finger, R.P., Krohne, T.U. (2019) Non-contact smartphone-based fundus imaging compared to conventional fundus imaging: a low-cost alternative for retinopathy of prematurity screening and documentation, Scientific reports. 9:19711.
[14] Wintergerst, M.W.M., Mishra, D.K., Hartmann, L., Shah, P., Konana, V.K., Sagar, P., Berger, M., Murali, K., Holz, F.G., Shanmugam, M.P., Finger, R.P. (2020) Diabetic retinopathy screening using smartphone-based fundus imaging in India, Ophthalmology. 127:1529-38.
[15] Wintergerst, M.W.M., Jansen, L.G., Holz, F.G., Finger, R.P. (2020) Smartphone-Based Fundus Imaging-Where Are We Now? Asia-Pacific Journal of Ophthalmology. 9:308-14.

The known methods and/or devices of the prior art have the following disadvantages:
(1) Existing AI models to detect optic disc edema do not provide immediate, on-device diagnostics without the need for an Internet connection. This is a disadvantage because it limits the use of existing AI models and makes their use difficult in rural or remote regions without clinical specialists or Internet connection.
(2) Existing AI models to detect optic disc edema do not follow the operational workflow of (i) helping the user navigate to the optic disc using forecasting algorithms, (ii) helping the user identify the optic disc using object detection algorithms, and (iii) classifying the optic disc edema using classification algorithms. This is a disadvantage because it limits the use of the AI models to clinical specialists who are less likely to need the support of AI models to navigate to, identify, and classify the optic disc.
(3) Existing retinal diagnostics using conventional clinical devices are expensive, time consuming, and require clinical specialists that are not easy to access, and are not available to everybody. This is a disadvantage because it adds equipment, personnel, and transportation costs to the diagnosis pipeline.
(4) Existing AI models to detect optic disc edema are not built into a smartphone or tablet application that can be used by everybody. This is a disadvantage because it adds barriers and costs for end users to access the AI models.
(5) Data used to train existing models are collected and stored in centralized databases. This is a disadvantage because it requires administrative clearances (e.g., data sharing agreements) and poses a risk to confidentiality of medical data (e.g., sharing astronaut medical data with other space agencies).

Altogether, there is a critical need for novel, cost-saving approaches to mobile-based retinal diagnostics. This invention declaration helps to provide non-specialist users with the ability to perform retinal fundoscopy on a variety of mobile devices at low cost.

According to the first aspect, the current invention is directed to a mobile device for real-time retinal diagnosis.

The mobile device according to the invention comprises a capturing module configured to capture a number V of images of a retina of a patient, an object detection module configured to calculate an object detection score for the presence of an optic disc, and to calculate the location of the optic disc for each of the number V of images with a trained neural network for the detection of an optic disc, an extraction module configured to extract a partial image area of the optic disc in each of the retinas of the patient based on the previously calculated location of the optic disc in each of the images, a classification module configured to forecast the presence or absence of optic disc edema for each of the extracted partial image areas containing the optic disc with a trained convolutional neural network for the identification of optic disc edema, and a predictor module configured to calculate an overall prediction for the presence of optic disc edema for the patient, wherein V is an integer greater than 1.

According to the invention, the optic image data/images can be generated using a digital camera of a smartphone. The image data can also be formed by a single frame of a video captured by the image sensor of a digital camera or a mobile phone such as the camera sensor of any mobile device such as specifically a smartphone. In general, the image captured by the mobile device can be generated by an optic image sensor such as a CCD or CMOS sensor, with a dedicated lens assembly for capturing the retina of a patient. Such a lens assembly can be adapted to be attached to any standard mobile device such as an Apple or Android smartphone or tablet comprising a digital camera. Prior art lens assemblies are already known that can be attached in front of such a camera sensor of a mobile device such as a smartphone or tablet. Such lens assemblies are known and commercially available for example distributed under the trade names: "D-Eye, oDocs nun, Heine iC2, Welch Allyn iExaminer, Volk iNview".

The classification module according to the current invention can be configured to calculate a probability score for the presence of optic disc edema for each of the extracted partial image areas with a trained convolutional neural network for the identification of optic disc edema.

Further, preferably the predictor module is configured to categorize the probability scores of the partial image areas into two discrete classes, wherein the probability scores above a set threshold are classified as optic disc edema and the probability scores equal or below the set threshold are classified as no optic disc edema and further configured to compute an overall aggregated prediction using the majority class of the classified partial image areas as overall prediction for the presence of optic disc edema.

Preferably, the mobile device can further comprise a selection module configured to select a preselected number W of captured images from the number V of images by selecting the number W of images with the highest object detection score (calculated by the object detection module) and configured to discard the remaining number of N-W of the images, wherein the selected W images are provided to the classification module. The number W is an integer lower or equal to the number V.

The capturing module according to the current invention can be further configured to capture and to display a live video stream formed by a plurality of consecutively captured images and further configured to provide an indication of the approximated location of the optic disc in each of the captured images based on computer vision techniques.

Preferably, the mobile device further comprises a manual selection module configured to display each of the captured images on a display to a user and further configured to discard captured images upon manual selection by the user.

According to a second aspect, the current invention provides a computer implemented method for real-time retinal diagnosis comprising the steps of:
a) receiving a number V of images of a retina of a patient;
b) calculating an object detection score for the presence of an optic disc and calculating the location of the optic disc for each of the images of step a) with a trained neural network for the detection of an optic disc;
c) extracting a partial image area of the optic disc in each of the images of the retina of the patient based on the calculated location of the optic disc;
d) calculating a probability score for the presence of optic disc edema for each of the extracted partial image areas with a trained convolutional neural network for the identification of optic disc edema; and
e) categorizing the probability scores of the partial image areas into two discrete classes, wherein the probability scores above a set threshold are classified as optic disc edema and the probability scores equal or below the set threshold are classified as no optic disc edema and computing an overall aggregated prediction as the majority class of the classified partial image areas.

The computer implemented method according to the present invention can preferably comprise after step b) and prior to step c) the additional step of:
b.1) selecting a preselected number W of captured images from the number V of the images by selecting the number W of images with the highest object detection score for the presence of an optic disc and discarding the remaining number of N-W of the images and providing the selected number W of images for step c).

More preferably the computer implemented method further comprises prior to step a) the additional step when the optic disc is not located on the image:
capturing and displaying a live video stream formed by a plurality of consecutively captured images and further providing an indication of the approximated location of the optic disc in each of the captured image data sets based on computer vision techniques.

Optionally, the computer implemented method according to the invention further comprises after step a) and prior to step c) the additional step:
displaying each of the received images on a display to a user and allowing the user to discard captured images upon manual selection by the user.

According to a third aspect, the current invention provides a computer implemented method for training a neural network for the detection of an optic disc, wherein the neural network is based on a pre-trained SSD Mobilenet V2 object detection module, wherein the method comprises the steps:
- providing a data set comprising a plurality of annotated images of the retina of several patients, wherein the annotation for each image contains information if an optic disc is present and the coordinates of a bounding box (which can be a square or a polygon with horizontal and vertical lines and offset margins to the optic disc) delimiting the optic disc;
- comparing several object detection models based on a K-Fold Cross Validation using patient/eye splitting or patient-only splitting;
- fine-tuning the best model (which was for this task a pre-trained SSD Mobile net V2 object detection model) according to some metric such as mean average precision, recall, speed, or a combination thereof. This can be done utilizing the holdout method based on a patient/eye or a patient-only splitting strategy on the previous data set. The SSD Mobile net V2 model is preferably configured with the following settings:
   - L2 regularizer
   - Dropout set to 0.5
   - Aspect ratios in the range [0.5, 1, 2],
   - weighted sigmoid local classification loss
   - momentum optimizer with a cosine decay learning rate
   - maximum number of localizations set to 1.

According to the fourth aspect of the current invention, a computer implemented method for training a convolutional neural network, CNN, for the identification of an optic disc edema is provided, wherein the CNN comprises a final output layer with a single neuron with a sigmoid activation function comprising:
- providing a data set with a plurality of annotated images of an optic disc of a patient, wherein the annotation contains information if an optic disc edema is present or absent in the image,
- preferably using an Adam optimizer with a batch size set to 32 and an L1_L2 regularizer for 100 epochs and the holdout method based on a patient/eye splitting or a patient-only splitting strategy,
- more preferably obtaining the architecture of the CNN by using a Bayesian hyper-parameter optimization algorithm with the objective of minimizing the validation loss, for a certain number of trials and several executions per trial.

According to a fifth aspect, the current invention provides a further computer implemented method based on computer vision for forecasting the approximated location of the optic disc comprising the following steps:
- providing an image of the retina of a patient,
- converting the image to grayscale,
- using a Sato ridge extraction technique to highlight the vessel structure of the retina, preferably using a set of sigmas in the range of [1,5],
- applying a Gaussian blur;
- applying a binary threshold to highlight white pixels with a grayscale above a defined first threshold,
- removing blocks of contiguous white pixels with an area below a defined second threshold,
- removing potential lens artifacts by using a HoughCircles transformation and removing potential circles,
- considering the two blocks of white pixels with the largest areas as the two biggest veins of the retina of the patient,
- approximating the two biggest veins with two lines using the HoughLines transformation on the two blocks of white pixels with the largest areas, and
- calculating the intersection point of the two lines as the approximated position of the optic disc.

According to a further aspect the current invention provides a data processing apparatus comprising means for carrying the computer implemented method according to the second aspect of the current invention.

According to yet another aspect the current invention provides a computer program comprising instructions which, when the program is executed by a computer, cause the computer to carry out the computer implemented method according to the second aspect of the current invention. Furthermore, a computer-readable medium is provided according to the invention having stored thereon the computer program according to the previous aspects of the invention. Furthermore, the current invention provides the ability for the computer implemented methods to learn, improve and iterate using a Federated Learning paradigm.

The features that distinguish the invention from the corresponding disadvantages of the existing prior art can be listed as follows:
The computer implemented method and/or mobile devices according to the current invention are able to detect optic disc edema immediately, with on-device diagnostics without the need for an internet connection. This is an advantage because they can be used in rural or remote regions where clinical specialists or Internet connection may be unavailable.

The figure 2 shows a screenshot of the object detection module of the mobile device according to the current invention operating in real-time.

The figure 3 depicts a screenshot of a classification module configured to forecast the presence or absence of optic disc edema for each of the extracted partial image areas containing the optic disc with a trained convolutional neural network for the identification of optic disc edema operating in real-time on the mobile device.

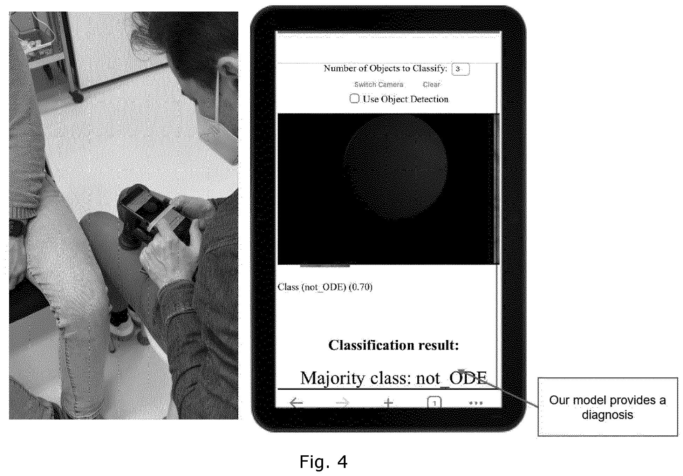

Figure 4. Screenshot of edema classification model operating in real-time (image credit: Franco Terranova).

According to the invention, a plurality of dedicated trained neural networks are used within the mobile device and/or the computer implemented method of the present invention. The computer implemented method for the real-time retinal diagnosis follows an operational workflow to (i) help the user navigate to the optic disc using forecasting algorithms based on computer vision techniques, (ii) help the user identify the optic disc using object detection algorithms with a trained neural network for the detection of an optic disc, and (iii) help the user classify the optic disc using classification algorithms with a trained convolutional neural network for the identification of optic disc edema. This is an advantage because it allows non-specialists to perform checks to navigate to, identify, and classify optic discs with minimal training.

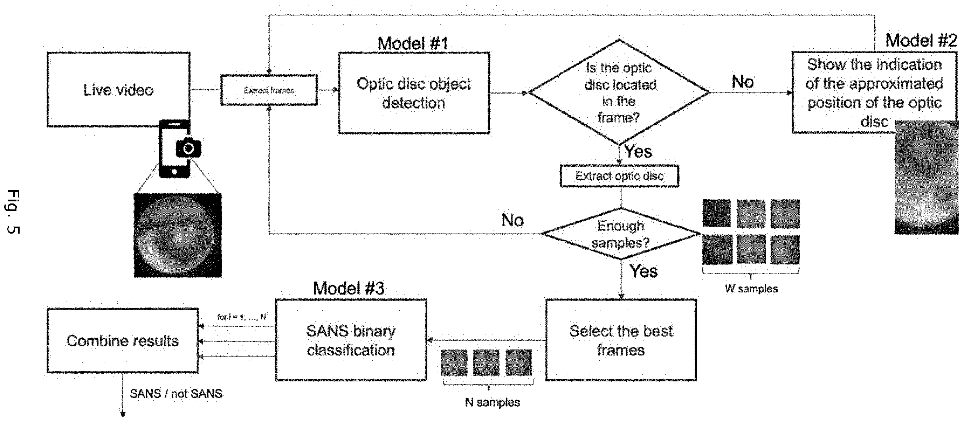

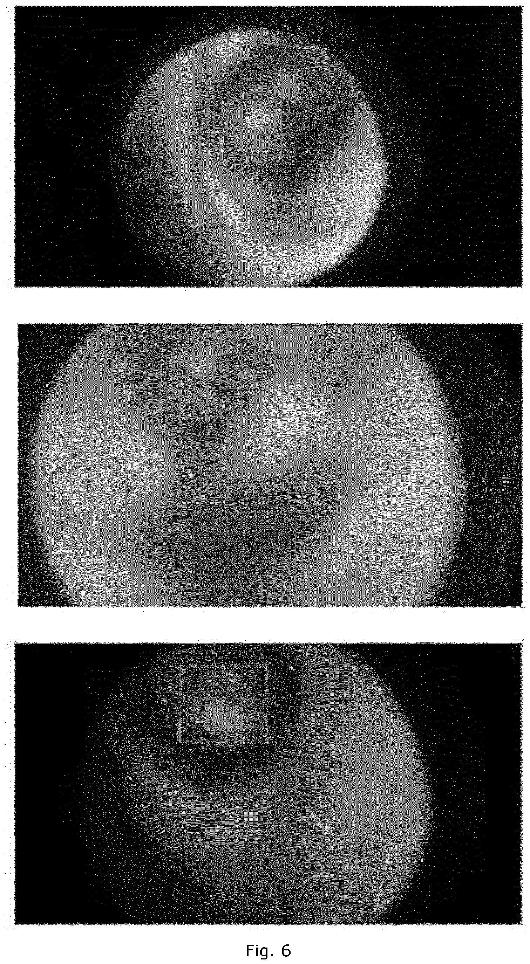

The figure 5 shows a schematic overview of an exemplary embodiment of the current invention of the navigation, detection, and classification operational workflow.

The software can be installed on mobile devices with cameras and used quickly by non-specialists. This advantage minimizes the equipment, personnel, and transportation costs associated with a diagnosis pipeline.

The AI models/trained neural networks can be deployed on smartphones and tablet applications, which can be used by everybody. This is an advantage because it reduces barriers and costs for anyone to access and use the AI models. A new artificial intelligence approach called "federated learning" can be used to increase the number of test subjects included in the training dataset by training the models locally and only sharing the weights of the neural network. This is an advantage because it enriches the size of the training dataset without the need for administrative clearances (e.g., data sharing agreements) and risks to confidentiality of medical data that are characteristic of the state of the art.

The user loads the application (computer implemented method), initiates the navigation/detection models, and performs fundoscopy. When the optic disc is not in frame, the navigation model predicts where the optic disc should be, so the user can move the device there. When the optic disc is in frame, the detection model draws a bounding box around the optic disc, captures frames of the optic disc and corresponding object detection scores, shows these frames to the user, allows the user to delete low quality frames alternatively bad quality images can be automatically deleted/discarded on the basis of the value of the object detection score and for example to discard images with a score value under a preset threshold value, and allows the user to classify the remaining frames to check if optic disc is present. The current inventions provide a computer implemented method, which contains a workflow and trained neural models, for navigation, detection, and classification of the optic disc as being normal or pathologic.

Altogether, the key invention features are AI models/trained neural networks within the claimed computer implemented method according to the second aspect of the invention and/or the claimed mobile device according to the first aspect of the current invention for optic disc navigation, detection, and classification, that operate in real-time and do not require an Internet connection. This can help non-specialist clinicians detect retinal changes (e.g., optic disc edema), which can result from intracranial pressure (ICP) changes (e.g., after head injuries, neurological issues). This can help determine the most appropriate course of action and avoid fatal outcomes.

The end users of the claimed mobile device include clinicians in emergency medicine, neurology, mobile clinics, home care, and remote areas who may otherwise not have access to a specialist physician (e.g., ophthalmologist or neurologist). This helps non-specialist clinicians detect optic disc pathologies that they may otherwise miss.

Potential areas of commercial application are listed below:
(1) Home care. Home care providers could use this product in the homes of their patients, as it could be easily installed on mobile devices for use in home medical kits.
(2) First response. First responders could use this product to check for possible head injuries or signs of intracranial pressure, as it could be easily installed on mobile devices for use in first-response kits.
(3) Remote medicine. Non-specialist clinicians could use this product when working in remote regions (e.g., Médecins Sans Frontières).
(4) Sports. Sports that involve physical contact where head injuries are a risk could install this software on their team's mobile devices, to check for possible head injuries.
(5) Medical education. Students can use this software to perform fundoscopies as part of their medical education.

### 1. Overall Application

The computer implemented method according to the second aspect of the invention can have the following workflow (see figure 5):
- An object detection model will be used to try to locate in each frame the position of the optic disc.
- Images of the optic disc will be extracted once located.
- A position forecaster model will be used when the optic disc is not present in the frame.
- Once enough samples of the optic disc are saved, the application will select the best frames using the object detection score.
- A binary classification will be applied on each of the optic disc images, in order to identify the optic disc edema.
- Results will be combined using a majority approach (majority class of the individually classified samples).

The value of N can be set by the user in the application and N can be lower or equal to W.

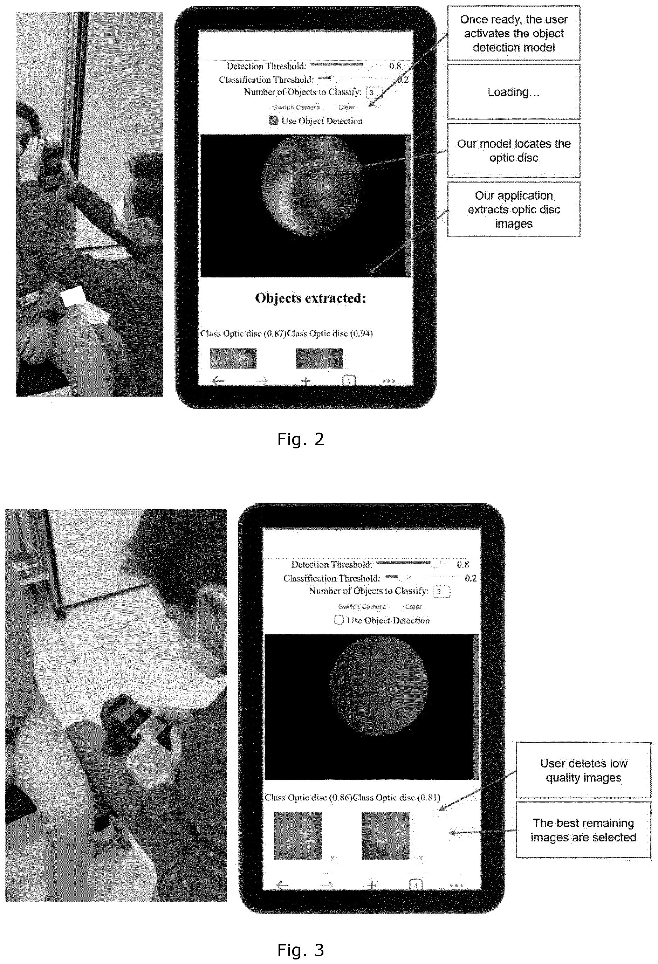

W is a number that is determined by the moment the user clicks the classification button. Once the user decides it is enough for data collection and it is time for classification, the number of samples collected will represent W. Before deciding to move toward the classification stage, the user can remove some images that may look like low-quality images in order to help the next stage on selecting the best frames (figure 2 and 3).

### 2. Model #1: Object detection module

The idea behind the first model is to train a neural network able to detect the optic disc given a retinal image using object detection techniques.

The input of the model will be an image or frame of a video and the output will be the coordinates of the bounding box surrounding the optic disc and the object detection score associated.

Tensorflow and the TensorFlow Object Detection API have been used in order to fine-tune different well-known object detection models (https://github.com/tensorflow/models/blob/master/research/object_detection/g3doc/tf2_detection_zoo.md), pre-trained on Common Objects in a common context (COCO) datasets. The need for transfer learning (fine-tuning pre-trained models) was mainly due to the presence of a few training samples.

### 2.1. Dataset

The dataset used by the object detection model has been obtained starting from the videos available and extracting equally spaced frames.

Among the frames extracted, at most 7 frames have been labeled for each video, in order to weight the training in the same manner.

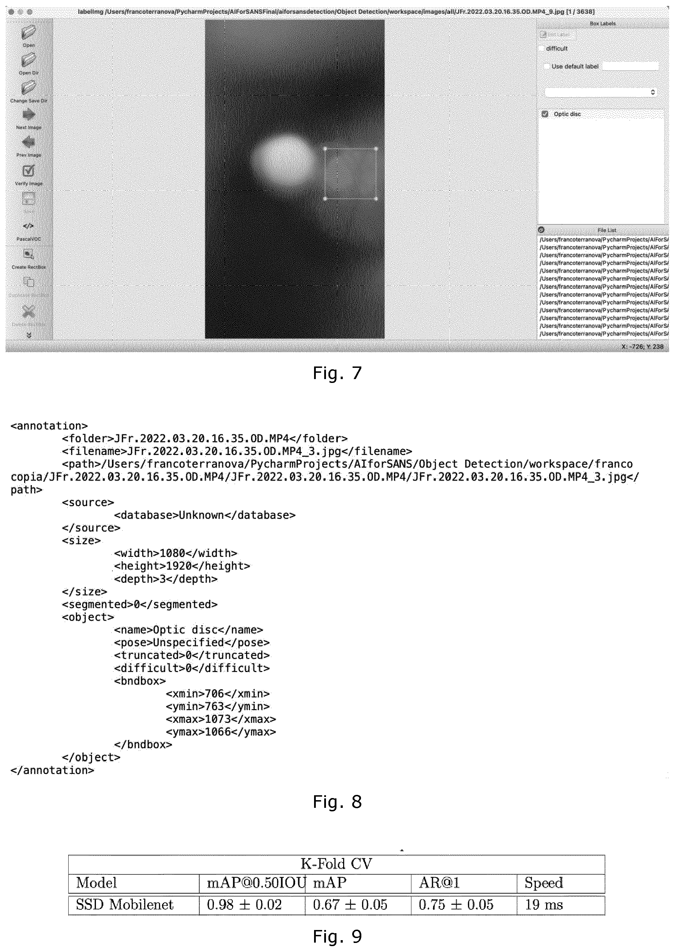

We manually annotated 3638 images using the labelImg tool, which provides an output for each image an XML file in PASCAL VOC format, which is an annotation file containing information about the bounding box coordinates and the associated class (see figure 7). Figure 7 shows an example of annotation of an image with labelImg tool and the corresponding XML file PASCAL VOC format.

### 2.2. Training of the object detection module/ of neural network for detection of the optic disc

A K-Fold Cross Validation (K-Fold CV) comparison using patient/eye pair splitting strategies and a number of folds set to 10 has been performed to assess the best pre-trained model to use. To evaluate the different networks, at each fold the networks were compared after 10k iterations.

The mean average precision and average recall were the main metrics used to compare the models, together with speed.

The SSD Mobilenet V2 object detection model architecture reached good performances on the test set and very good performances in terms of speed, and this is why it has been used for this study.

It is an efficient architecture introduced by Google (using depthwise and pointwise convolutions). It can be used for classification purposes, or as a feature extractor for other tasks (i.e. detection).

After a K-Fold CV for comparing models, SSD MobileNet underwent its final round of retraining utilizing the holdout method. Early stopping was suggested after approximately 50,000 iterations of training. The model was configured with the following settings:
- L2 regularizer
- Dropout set to 0.5
- Aspect ratios in the range [0.5, 1, 2], typical for our dataset
- Weighted smooth L1 localization loss
- Weighted sigmoid local classification loss
- A localization weight doubled with respect to the classification weight, mainly due to the presence of a single class and a harder localization task
- Momentum optimizer with a cosine decay learning rate
- A maximum number of boxes/localizations properly set to 1 for our task The other hyper-parameters of the model are less relevant for this task and have been used with their default values. (see figure 9)

### 3. Model #3: Classification Module/ convolutional neural network for the identification of optic disc edema

Convolutional Neural Networks (CNNs) are a type of neural network used primarily for image-processing tasks such as image classification.

This type of network has been used for the classification task, leveraging the Keras library for training the model.

For binary classification purposes, a CNN uses a final output layer with a single neuron with a sigmoid activation function. The sigmoid function produces a value between 0 and 1, which can be interpreted as the probability of the input image belonging to one class or the other (optic disc edema (ODE) or not ODE).

The input of the model will be an image of a cropped optic disc (cropped using the coordinates forecasted by the object detection model) and the output will be a score representing the probability to belong to the class ODE and not ODE. Identifying ODE can be seen as equivalent for identifying SANS.

If the probability is greater than a threshold value, the input image is classified as belonging to the first class; otherwise, it is classified as belonging to the second class.

### 3.1. Dataset

The dataset used for the analysis has been generated using videos coming from the Helios University Clinic Wuppertal, providing videos of cases of ODE, and DLR bed rest studies, providing videos of cases without ODE.

The object detection model previously shown has been applied to the videos in order to generate the dataset needed for the classification phase.

The approach used involved splitting each video into 10 portions, analyzing each portion separately using object detection, and saving the optic disc with the highest object detection score from each portion. This resulted in a maximum of 10 frames per video, from which the best 3 frames were selected. This approach ensured that training was weighted equally for each video, and helped to remove noisy images such as blurry frames.

A stratified split by patient/eye pair has been used, in order to ensure that all batches of images of the same eye will end up in the same dataset portion in the holdout method.

Using this technique, the dataset was divided into a training set (70%), a validation set (15%), and a test set (15%).

The first problem encountered in the classification task was the presence of a highly imbalanced dataset, typical for medical problems that may have few samples for the positive class. To deal with this problem, class weights are introduced in the loss function by assigning a higher weight to the loss encountered by the samples associated with the minority class. As a result, instead of a typical binary cross-entropy loss function used for binary classification, a weighted binary cross-entropy has been used as a loss function.

### 3.2. Training

The Adam optimizer has been used during training using 224x224 size images with pixels normalized, a batch size set to 32 and a L1_L2 regularizer for 100 epochs.

The architecture of the network used has been obtained using hyper-parameter optimization with the *keras_tuner* library.

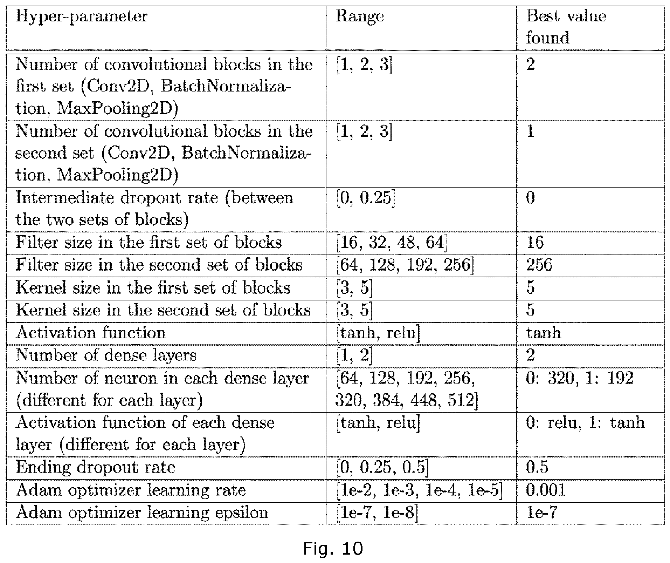

The Bayesian hyper-parameters optimization algorithm has been used with the objective of minimizing the validation loss, with a maximum number of trials set to 50 and 2 executions per trial. Figure 10 Hyper-parameters range and best value found.

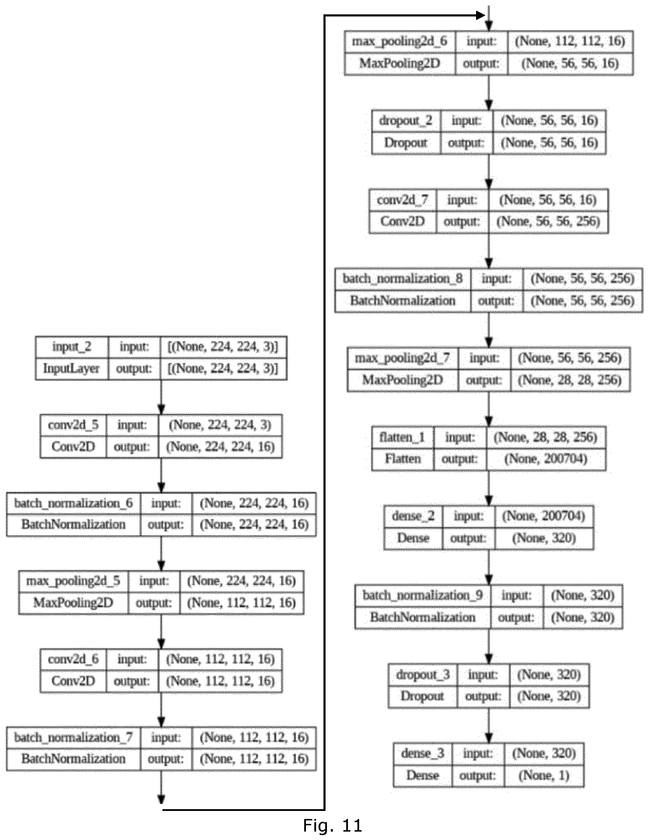

The best value found represents the value used for each of the hyper-parameters of the hyper-optimized model used for classification. Figure 11 Hyper-optimized model architecture.

The EarlyStopping callback interrupted training when accuracy stopped improving on the validation set for at least a certain number of epochs, challenging overfitting. The patience parameter has been set to 5.

The ReduceLROnPlateau callback was used to monitor the model's validation loss and reduce the learning rate (LR) when the validation loss stopped improving, an effective strategy to escape local minima. The patience parameter has been set to 3.

### 3.3. Testing

Performances obtained on the test set using the holdout method are shown in this section.

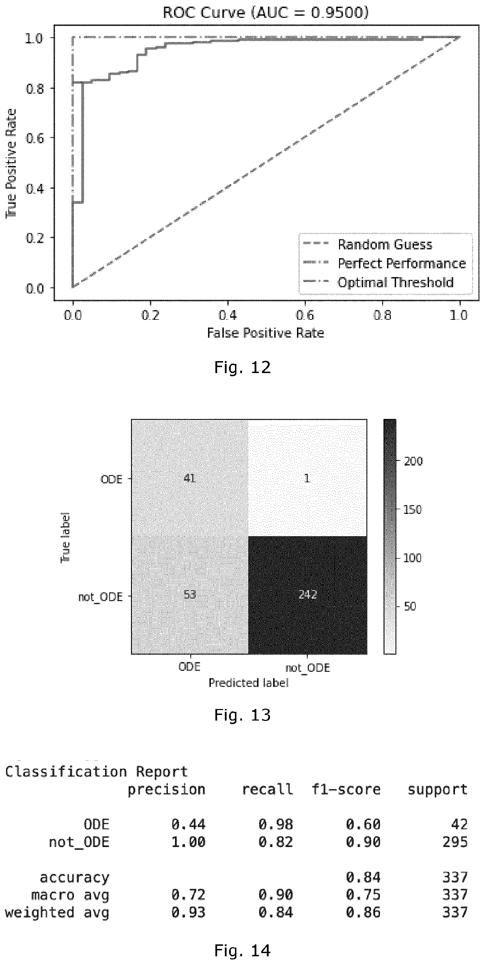

To determine the optimal threshold to use for the output of the last sigmoid neuron, the Receiver Operating Characteristic (POC) curve has been used. Using the difference between the true positive rate and false positive rate as the objective to maximize, the optimal threshold has been obtained for this network. Figure 12 POC Curve for the classification model used to determine the optimal threshold. Figure 13 Confusion Matrix of the classification model. Figure 14 Classification report of the classification model.

### 4. Model #2: Optic Disc Position Forecasting Model

The optic disc position forecaster is a model consisting of a series of computer vision techniques able to predict the point where the optic disc is located. This model will take the image as input and will output the point representing the position of the optic disc.

One way to locate the optic disc in the eye is to look for the bifurcation of veins. As the veins travel through the retina, they branch off into smaller vessels, forming a pattern known as the venous tree. At the optic disc, the veins converge and exit the eye as the central retinal vein.

Following the previous hint, a series of computer vision techniques can be applied to retinal images in order to highlight the veins and find their bifurcation.

### 4.1. Methodology

The computer vision pipeline used to forecast the optic disc position has been implemented using the OpenCV library and it mainly relies on the use of the ridge extraction technique.

Ridge extraction is a process used in image processing to identify and extract the ridges or prominent features of an image. In the context of retinal images, ridge extraction can be used to highlight the blood vessels and other structures in the retina.

Comparing different ridge extraction techniques, Sato seemed to be the technique with the best general visual performances for this task, using a set of sigmas in the range [1,5].

The computer vision approach implemented follows the subsequent pipeline for each frame.

The image is converted to grayscale.
1. The Sato ridge extraction technique is used to highlight the vessel structure.
2. A Gaussian blur is used to reduce the amount of noise present in the image.
3. Binary thresholding is used in order to highlight white pixels with a grayscale value above a certain threshold.
4. Blocks of contiguous white pixels with an area below a certain threshold are removed (hopefully not vessels).
5. Due to the presence of the contrast created by the leans, a circle is shown in the image while passing to the binary representation. These circles, if present, are identified using the HoughCircles transformation and removed properly.
6. In case the number of remaining white pixels is below a particular threshold, we assume no veins are contained in the frame, moving towards the next frame.
7. The two blocks of white pixels with the largest area (hopefully the two biggest veins) are considered for the next steps.
8. The two veins are approximated with lines using the HoughLines transformation.
9. Their intersection is considered as the approximated position of the optic disc.

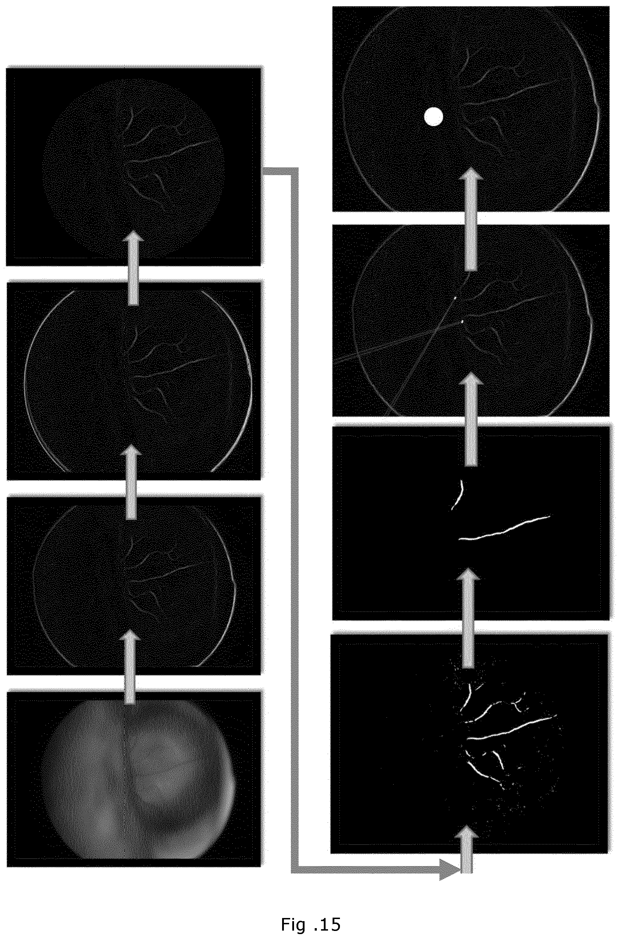

The thresholds used for each of these steps depend and were adapted based on the specific video on which the serious of techniques were applied. Figure 15 Pipeline of the Computer Vision sequence of instructions.

### 4.2. Combining predictions

During the execution of the model in each frame of a video, the forecasted positions can be properly combined in a smart manner.

During the live execution of the model, the contributions may give contrasting results that take into consideration the change of the optic disc's position and the possible presence of noise.

To take into consideration all these factors, the image space can be discretized, and the contributions can give a certain weight to a cell with linear decay in time.

Using this approach, the most voted cell can be used as the approximated position of the optic disc, reducing the presence of noise that may hardly be so high to affect the most voted cell, while the decay will take into consideration the changes in the position of the optic disc during the live execution of the video.

### 5. Federated Learning

One issue with deep learning is that a large dataset is typically required to attain statistical significance when tackling space medicine challenges. However, sharing medical data across agencies is limited by privacy concerns, and some agencies may be hesitant to share the data of their crew members. Federated learning (FL) is an emerging machine learning paradigm that can address these challenges by enabling decentralized training of AI models on local datasets, which are then aggregated into a global model without raw data sharing. This method increases the number of study subjects, dataset size, and accuracy of AI models, while also avoiding privacy and security risks and reducing energy consumption and communication costs. FL can be used to advance AI models to identify, monitor, and prevent Spaceflight Associated Neuro-ocular Syndrome (SANS) and improve space medicine and crew performance during long-duration spaceflight missions to the Moon and Mars. After decentralized training on local data at each site, the local model parameters are shared and aggregated into a cross-agency global model.

An architecture composed of a master and slaves can leverage a communication layer for exchanging the weight of the local networks and enable the paradigm.

## Claims

1. Mobile device for real-time retinal diagnosis comprising:
- a capturing module configured to capture a number V of images of a retina of a patient,
- an object detection module configured to calculate an object detection score for the presence of an optic disc and to calculate the location of the optic disc for each of the number V of images with a trained neural network for the detection of an optic disc,
- an extraction module configured to extract a partial image area of the optic disc in each of the images of the retina of the patient based on the previously calculated location of the optic disc in each of the images,
- a classification module configured to forecast the presence or absence of optic disc edema for each of the extracted partial image areas containing the optic disc with a trained convolutional neural network for the identification of optic disc edema, and
- a predictor module configured to calculate an overall prediction for the presence of optic disc edema for the patient.

2. Mobile device according to claim 1, wherein the classification module is configured to classify each of the partial image areas in two classes, forecasting a probability score for each image, such that if the probability is above a predefined threshold, the image is classified as optic disc edema, while if the probability score is equal or below the predefined threshold, the image is classified as not containing the optic disc edema, wherein the predictor module is configured to compute an overall aggregated prediction using the majority class of the classified partial image areas.

3. Mobile device according to claim 1 or 2, wherein the mobile device further comprises a selection module configured to select a preselected number W of captured images from the number V of images by selecting the number W of images with the highest object detection score and configured to discard the remaining number of N-W of the images, wherein the selected W images are provided to the classification module.

4. Mobile device according to any one of claims 1 to 3, wherein the capturing module is configured to capture and to display a live video stream formed by a plurality of consecutively captured images and further configured to provide an indication of the approximated location of the optic disc in each of the captured images based on computer vision techniques.

5. Mobile device according to any one of claims 1 to 4, wherein the mobile device further comprises a manual selection module configured to display each of the captured images on a display to a user and further configured to discard captured images upon manual selection by the user.

6. A computer-implemented method for real-time retinal diagnosis comprising the steps of:
a) receiving a number V of images of a retina of a patient;
b) calculating an object detection score for the presence of an optic disc and calculating the location of the optic disc for each of the images of step a) with a trained neural network for the detection of an optic disc;
c) extracting a partial image area of the optic disc in each of the images of the retina of the patient based on the calculated location of the optic disc;
d) calculating a probability score for the presence of optic disc edema for each of the extracted partial image areas with a trained convolutional neural network for the identification of optic disc edema; and
e) calculating an average probability score for the presence of an optic disc edema by calculating an average of the plurality of probability scores of step d) or
categorizing the probability scores of the partial image areas into two discrete classes, wherein the probability scores above a set threshold are classified as optic disc edema and the probability scores equal or below the set threshold are classified as no optic disc edema and computing an overall aggregated prediction as the majority class of the classified partial image areas.

7. The computer-implemented method of claim 6, wherein the method further comprises after step b) and prior to step c) the step:
b.1) selecting a preselected number W of captured images from the number V of the images by selecting the number W of images with the highest object detection score for the presence of an optic disc and discarding the remaining number of N-W of the images and providing the selected number W of images for step c).

8. The computer-implemented method of claim 6 or 7, wherein the method further comprises prior to step a) the additional step when the optic disc is not located on the image:
capturing and displaying a live video stream formed by a plurality of consecutively captured images and further providing an indication of the approximated location of the optic disc in each of the captured image data sets based on computer vision techniques.

9. The computer-implemented method of any one of claims 6 to 8, wherein the method further comprises after step a) and prior to step c) the additional step: displaying each of the received images on a display to a user and allowing the user to discard captured images upon manual selection by the user.

10. A computer-implemented method for training a neural network for the detection of an optic disc, wherein the neural network is based on a pre-trained SSD Mobilenet V2 object detection model, wherein the method comprises the steps:
- providing a data set comprising a plurality of annotated images of the retina of several patients, wherein the annotation for each image contains information if an optic disc is present and the coordinates of a bounding box, which can be a square or a polygon with horizontal and vertical lines and offset margins to the optic disc, delimiting the optic disc;
- comparing several object detection models based on a K-Fold Cross Validation using patient/eye splitting or patient-only splitting;
- fine-tuning the best model (which was for this task a pre-trained SSD Mobile net V2 object detection model) according to some metric such as mean average precision, recall or speed. This can be done utilizing the holdout method based on a patient/eye or a patient-only splitting strategy on the previous data set. The SSD Mobile net V2 model is preferably configured with the following settings:
• L2 regularizer
• Dropout set to 0.5
• Aspect ratios in the range [0.5, 1, 2],
• weighted sigmoid local classification loss
• momentum optimizer with a cosine decay learning rate
• maximum number of localizations set to 1.

11. A computer implemented method for training a convolutional neural network, CNN, for the identification of an optic disc edema, wherein the CNN comprises a final output layer with a single neuron with a sigmoid activation function comprising:
- providing a data set with a plurality of annotated images of an optic disc of a patient, wherein the annotation contains information if an optic disc edema is present or absent in the image,
- preferably using an Adam optimizer which a batch size set to 32 and a L1_L2 regularizer for 100 epochs and the holdout method based on a patient/eye splitting or a patient-only splitting strategy.
- more preferably obtaining the architecture of the CNN by using a Bayesian hyper-parameter optimization algorithm with the objective of minimizing the validation loss, for a certain number of trials and several executions per trial.

12. A computer implemented method for forecasting the approximated location of the optic disc comprising the following steps:
- providing an image of a retina of a patient,
- converting the image to grayscale,
- using a Sato ridge extraction technique to highlight the vessel structure of the retina, wherein preferably using a set of sigmas in the range of [1,5],
- applying a Gaussian blur;
- applying a binary threshold to highlight white pixels with a grayscale above a define first threshold,
- removing blocks of contiguous white pixels with an area below a defined second threshold,
- removing potential lens artefacts by using a HoughCircles transformation and removing potential circles,
- considering the two blocks of white pixels with the largest areas as the two biggest veins of the retina of the patient,
- approximating the two biggest veins with two lines using the HoughLines transformation on the two blocks of white pixels with the largest areas, and
- calculating the intersection point of the two lines as the approximated position of the optic disc.

13. A data processing apparatus comprising means for carrying out the method of any one of claims 6 to 9.

14. A computer program comprising instructions which, when the program is executed by a computer, cause the computer to carry out the method of any one of claims 6 to 9.

15. A computer-readable medium having stored thereon the computer program of claim 14.

16. The ability for the computer implemented method described in 1-15 to learn, improve, and iterate using a Federated Learning paradigm
